## Europäisches Patentamt
## European Patent Office
## Office européen des brevets

(19)

(11) Publication number: **0 002 098**
**B1**

(12)

# EUROPEAN PATENT SPECIFICATION

(45) Date of publication of patent specification: **12.08.81**

(51) Int. Cl.³: **C 07 C 17/38**

(21) Application number: **78300306.4**

(22) Date of filing: **21.08.78**

(54) Removal of perfluoroisobutylene.

(30) Priority: **10.11.77 US 850221**

(43) Date of publication of application:
**30.05.79 Bulletin 79/11**

(45) Publication of the grant of the European patent:
**12.08.81 Bulletin 81/32**

(84) Designated Contracting States:
**DE FR GB**

(56) References cited:
**US - A - 2 999 885**
**US - A - 3 004 075**
**US - A - 3 009 966**
**US - A - 3 321 383**
**US - A - 3 548 015**

**CHEMICAL ABSTRACTS, vol. 84, 4446g (1976)**
**Columbus, Ohio, USA**
**Y. Yoneya et al.: "Removal of octafluoroiso-**
**butene from hexafluoropropylene gas".**
**JOURNAL OF THE AMERICAN CHEMICAL**
**SOCIETY, vol. 79, page 1741—4, (1957)**

(73) Proprietor: **E.I. DU PONT DE NEMOURS AND COMPANY**
**Legal Department 1007 Market Street**
**Wilmington, Delaware 19898 (US)**

(72) Inventor: **Treat, Theodore Arthur**
**534 Lakewood Circle**
**Washington West Virginia 26184 (US)**

(74) Representative: **Myerscough, Philip Boyd et al,**
**J.A.Kemp & Co. 14, South Square Gray's Inn**
**London, WC1R 5EU (GB)**

Courier Press, Leamington Spa, England.

Removal of perfluoroisobutylene

This invention relates to removal of perfluoroisobutylene from a mixture of fluorine-containing compounds.

BACKGROUND OF THE INVENTION

Formation of perfluoroisobutylene occurs under a variety of conditions; for example, during heating of tetrafluoroethylene at 750°C or above, during pyrolysis of chlorodifluoromethane to produce tetrafluoroethylene and hexafluoropropylene as described in Halliwell U.S. Patent 3,306,940, during pyrolysis of other materials to make tetrafluoroethylene, such as pyrolysis of bromodifluoromethane (described in Canadian patent 686,106) or tetrafluoromethane (described in U.S. Patent 3,081,245), or during pyrolysis of tetrafluoroethylene to make hexafluoropropylene (described in U.S. 3,578,721; U.S. 3,873,630; and U.S. 2,785,138). The perfluoroisobutylene so formed is separated from the desired products by distillation at superatmospheric pressure along with a number of other fluorine-containing by-products. Because of the toxicity of perfluoroisobutylene, i.e., its harmful effect on test animals, it is desirable to convert it to a less toxic material prior to disposal.

Reaction of perfluoroisobutylene with aliphatic alcohols is known in the art, as evidenced by R.J. Koshar et al., JACS *79*, 1741 (1957). Although the JACS article reports successful reactions with several alcohols, including methanol, under neutral or slightly acidic conditions, strongly acidic conditions, in particular those resulting from the formation of hydrogen fluoride in side reactions, are stated to prevent continuing reaction between alcohol and perfluoroisobutylene. For this reason, removal of perfluoroisobutylene, especially from the by-products obtained during manufacture of tetrafluoroethylene and hexafluoropropylene, has heretofore been carried out by contacting the perfluoroisobutylene with sodium hydroxide in aqueous methanol. However, the use of sodium hydroxide in aqueous methanol has several disadvantages, namely, it reacts with other fluoroolefins which may be desired products, such as hexafluoropropylene, and it reacts with HCl and HF to form halide salts which foul the processing equipment and create waste disposal problems.

Accordingly, it is desirable to develop a procedure for removing toxic perfluoroisobutylene from a mixture of fluorine-containing compounds, especially compounds obtained as by-products during manufacture of tetrafluoroethylene and hexafluoropropylene, that is free of the disadvantages discussed above.

This invention provides a process for removing perfluoroisobutylene from a gaseous mixture of fluorine-containing compounds containing the same by contacting the said mixture with liquid methanol to convert the perfluoroisobutylene to a less toxic ether, characterised in that the said gaseous mixture is contacted with a liquid solution of hydrogen fluoride and/or hydrogen chloride in methanol, the solution initially containing over $10^{-7}$ moles/litre of hydrogen fluoride and/or hydrogen chloride.

A convenient, and preferred, procedure is to pass the methanol solution down a column while the mixture of fluorine-containing compounds, in gaseous form, is passed up the column in countercurrent flow. Another procedure is to bubble the gaseous mixture through the methanol solution. Still another procedure is to employ a closed vessel in a batch reaction.

Contact time is not critical and depends on how much perfluoroisobutylene is desired to be removed from the gaseous mixture.

The amount of the methanol that is employed is not critical, but to convert all the perfluoroisobutylene to the ether product, an excess of methanol over the amount of perfluoroisobutylene present should be employed.

Temperature and pressure are not critical, provided the methanol solution is liquid. The process may be carried out above or below atmospheric pressure, or, preferably, at atmospheric pressure. Preferably the contact is carried out at 20 to 100°C and at atmospheric pressure.

The reaction medium is acidic due to the initial presence in the methanol solution of over $10^{-7}$ moles/litre of HF and/or HCl. Additional HF may be formed by reaction of the methanol with the fluorine-containing compounds. The reaction medium remains acidic because HF and HCl each forms a high boiling azeotrope with methanol.

The initial HF and/or HCl concentration of the methanol solution preferably is between $10^{-7}$ moles/l and 2 moles/l.

The process may be carried out by batch or continuous procedures. The acidic methanol is ordinarily recycled in a continuous process.

The ether products of the reaction are methyl ethers, namely $(CF_3)_2CHCF_2OCH_3$ or $(CF_3)_2C{=}CFOCH_3$, and can be isolated or vented. The ethers are much less toxic than perfluoroisobutylene.

In the Examples which follow, High Boiling Product Mixture (HBPM) from the pyrolysis of chlorodifluoromethane was employed. The HBPM is so named because it boils higher than hexafluoropropylene, the desired product of the pyrolysis. The HBPM is a mixture of saturated and unsaturated halocarbons and hydrohalocarbons containing perfluoroisobutylene.

EXAMPLE 1

The following was charged to a 75 ml, dry ice cooled, stainless steel cylinder:

20 ml (30g) HBPM containing about 7 mole% perfluoroisobutylene

19 ml (15g) methanol containing 4.1 m/l HF (which resulted in a methanol concentration of 12 moles/liter and an HF concentration of 2 moles/liter).

The cylinder was then shaken and immersed in a water bath initially at 30°C to warm it to about 22°C at equilibrium. The concentration of perfluoroisobutylene (PFIB) was measured by gas chromotograph at the times indicated below:

15 min after immersion — 49% of original PFIB was present

47 min after immersion — 9.2% of original PFIB was present

80 min after immersion — 2.4% of original PFIB was present.

EXAMPLE 2

Through a 2.2 cm diameter by 30 cm scrubbing column packed with 4.8 mm stainless steel Heli-pak from Podbelniak Inc. was passed HBPM gas containing about 10 mole% perfluoroisobutylene at a rate of 160 ml/min. A scrubbing solution of methanol containing 12 mole/liter HCl was continuously recycled through the column. The methanol solution was continuously recycled at 25°C. The concentration of perfluoroisobutylene (PFIB) in the gas was measured at the entrance and at the exit of the column by gas chromatography. The concentration of PFIB present in the gas at exit was only 1.1% of the amount present in the gas introduced into the column.

## Claims

1. A process for removing perfluoroisobutylene from a gaseous mixture of fluorine-containing compounds containing the same by contacting the said mixture with liquid methanol to convert the perfluoroisobutylene to a less toxic ether, characterised in that the said gaseous mixture is contacted with a liquid solution of hydrogen fluoride and/or hydrogen chloride in methanol, the solution initially containing over $10^{-7}$ moles/liter of hydrogen fluoride and/or hydrogen chloride.

2. A process according to claim 1 characterised in that the contact is carried out at a temperature of 20 to 100°C and at atmospheric pressure.

3. A process according to claim 1 or 2 characterised in that the initial concentration of hydrogen fluoride and/or hydrogen chloride in the said solution is between $10^{-7}$ moles/liter and 2 moles/liter.

## Revendications

1. Procéde d'élimination du perfluoroisobutylène à partir d'un mélange gazeux de composés fluores le contenant, par mise en contact du mélange avec du méthanol liquide pour convertir le perfluoroisobutylène en un éther moins toxique, caractérisé en ce que l'on met le mélange gazeux en contact avec une solution liquide de fluorure d'hydrogène et/ou de chlorure d'hydrogène dans du méthanol, la solution contenant initialement plus de $10^{-7}$ mole/litre de fluorure d'hydrogène et/ou de chlorure d'hydrogène.

2. Procédé selon la revendication 1, caractérisé en ce que l'on effectue le contact à une température de 20 à 100°C et à la pression atmosphérique.

3. Procédé selon la revendication 1 ou 2, caractérisé en ce que la concentration initiale de fluorure d'hydrogène et/ou de chlorure d'hydrogène dans la solution est comprise entre $10^{-7}$ et 2 moles/litre.

## Patentansprüche

1. Verfahren zum Entfernen von Perfluorisobutylen aus einer dieses enthaltenden gasförmigen Mischung fluorhaltiger Verbindungen, indem man die genannte Mischung mit flüssigem Methanol kontaktiert, um das Perfluorisobutylen in einen weniger toxischen Äther umzuwandeln, dadurch gekennzeichnet, dass die genannte gasförmige Mischung mit einer flüssigen Lösung von Fluorwasserstoff und/oder Chlorwasserstoff in Methanol kontaktiert, wobei die Lösung zu Anfang mehr als $10^{-7}$ Mol/l an Fluorwasserstoff und/oder Chlorwasserstoff enthält.

2. Verfahren nach Anspruch 1, dadurch gekennzeichnet, dass der Kontakt bei einer Temperatur von 20 bis 100°C und bei Atmosphärendruck durchgeführt wird.

3. Verfahren nach Anspruch 1 oder 2, dadurch gekennzeichnet, dass die anfängliche Konzentration an Fluorwasserstoff und/oder Chlorwasserstoff in der Lösung zwischen $10^{-7}$ und 2 Mol/l liegt.